# EUROPEAN PATENT SPECIFICATION

(11) **EP 1 210 317 B1**
(45) Date of publication and mention of the grant of the patent: **04.01.2006**
(21) Application number: 00959197.5
(22) Date of filing: 08.08.2000
(51) Int. Cl.: C07C 51/43, C07C 51/41, C07C 63/14, C07C 63/38

(54) **METHOD FOR REDUCTION OF POTASSIUM IN AN INTEGRATED PROCESS FOR THE PRODUCTION OF 2,6-NDA**
VERFAHREN ZUR VERRINGERUNG DES KALIUMS IN EINEM INTEGRIERTEN PROZESS ZUR HERSTELLUNG VON 2,6-NAPHTHALENEDICARBONSÄURE
PROCEDE DE REDUCTION DU POTASSIUM DANS UN PROCESSUS INTEGRE POUR LA PRODUCTION DE 2,6-NDA

(30) Priority: 30.08.1999 US 151602 P
(43) Date of publication of application: 05.06.2002
(73) Proprietor: Mossi & Ghisolfi International S.A., 1528 Luxembourg (LU)
(72) Inventor: BLACKBOURN, Robert, L., Houston, TX 77095 (US); JUNE, Raymond, L., Houston, TX 77079 (US)
(74) Representative: Gerbino, Angelo
(86) International application number: PCT/US2000/021687
(87) International publication number: WO 2001/016083

(56) References cited:
- EP-A- 0 672 644
- GB-A- 1 531 404
- US-A- 5 175 354
- US-A- 5 728 870
- JOHN WILEY AND SONS: "Encyclopedia of chemical technology" WILEY-INTERSCIENCE * page 883 *

## Description

### Technical Field

This invention is generally related to the purification of aromatic dicarboxylic acids, especially 2,6-naphthalene dicarboxylic acid according to the claims. More particularly, this invention is related to a practical method of reducing potassium in the final product to acceptable levels for subsequent polymerization.

### Background Art

Polymer grade aromatic dicarboxylic acids are important as starting materials for a number of polyester fibers, polyester films, resins for bottles and containers, and the like. Naphthalene dicarboxylic acids, especially 2,6-naphthalene dicarboxylic acid (hereafter referred to as 2,6-NDA) are starting materials for polyethylene naphthalates, which can also be employed in the manufacture of fibers, films, and resins. Presently in the art, it is very difficult to produce polymer grade 2,6-NDA and only dimethyl 2,6-naphthalene dicarboxylate, the methyl ester of the more desirable 2,6-NDA is commercially available for use in making polymers such as polyethylene naphthalate.

When aromatic dicarboxylic acids are produced by a disproportionation reaction, such as described in U.S. 2,823,231. and U.S. 2,849,482, for example, where an alkali metal salt of a mono- or dicarboxylic acid is disproportionated to produce isomers of the salt of the desired dicarboxylic acids, the product often retains undesirable amounts of the alkali metal, most often potassium.

The goal of a number of processes for purification of aromatic dicarboxylic acids, and 2,6-naphthalene dicarboxylic acid in particular, is to reduce these alkali metals along with other impurities to the lowest levels possible, but there is still a need in the art for methods of reducing impurities and alkali metals to levels acceptable for use in polymers.

Methods of purification of aromatic dicarboxylic acids, especially 2,6-naphthalene dicarboxylic acid are known in the art, e.g. in US-A-5 728 870 and US-A-5 175 354. Where 2,6-NDA is produced by disproportionation, common methods for purification include filtration, acidification and crystallization. See, for example, U.S. 2,849,482; 3,631,096; 3,671,578; and 3,952,052. It is possible to remove significant amounts of color bodies and impurities, but it is still difficult to obtain polymer grade 2,6-NDA.

Currently in the art the most common process for making 2,6 NDA starts with relatively expensive o-xylene and butadiene feedstocks, as discussed, for example, in U.S. 5,510,563 and U.S. 5,329,058 and incurs substantial yield losses of these starting materials. Following the synthesis and purification of 2,6 dimethylnaphthalene (2,6 DMN), 2,6 DMN is oxidized to produce crude NDA product which forms as a solid with impurities trapped within. Therefore, in such processes, esterification to naphthalene dicarboxylate (NDC) is necessary to eliminate the impurities, as discussed in US 5,254,719 and 4,886,901. Direct purification of the crude NDA via hydrogenation has been suggested by US 5,292,934, but requires a difficult and expensive high temperature hydrogenation in the presence of a solvent. Another proposed purification scheme requires the use of nitrogen containing species. (See U.S. 5,770,764 and U.S. 5,859,299). Crystal size and morphology is important in either case, whereas the novel process disclosed herein can optionally avoid the issue of controlling particle size of the final product.

Currently NDC is commercially available, but NDA is not, presumably because of the difficulty of producing polymerization grade NDA without esterifying to NDC. Ideally, if NDA were available commercially at a competitive price, NDA would be preferred over NDC as the starting monomer for PEN. Alternative routes to NDA based on the rearrangement reaction have been plagued with difficulties associated with handling solids, the inefficient recycling of potassium, and ineffective integration from feedstock through final product. Although various improvements have been suggested over the years, there is still a distinct need in the art for an economical, integrated process for producing polymer grade 2,6-NDA, the preferred monomer for the production of polyethylenenaphthalate (PEN). Copending U.S. Application Serial No. 60/151,577 discloses a novel integrated process for producing the preferred 2,6-naphthalene dicarboxylic acid.

US-A-1 531 404 discloses a process for the production of terephthalic acid of high purity.

It would represent a distinct advance in the art if a method were available to reduce the levels of alkali metals, particularly potassium in product 2,6-NDA to levels which are acceptable for polymerization to polyethylene naphthalate.

### Disclosure of the Invention

In accordance with the foregoing, the present invention provides a method according to claim 1 which follows.

In the preferred embodiment, the invention makes it possible to reduce the level of potassium in product 2,6-naphthalene dicarboxylic acid to less than 50 ppm.

### Detailed Description of the Invention

In the preferred embodiment the aromatic dicarboxylic acid which is treated in the present invention is 2,6-NDA produced by a process which incorporates disproportionation of an alkali metal salt of a mono- or dicarboxylic acid.

Following disproportionation the crude product is in the form of an isomer of a dialkali salt of 2,6-NDA. Salts formed by the reaction can be transformed into the corresponding acids by introducing carbon dioxide into the solution and then separating the free acids from the acidified solution. The salt mixture produced by the reaction may also be transformed directly into derivatives of the acids, such as, for example, their esters or halides, and these derivatives can be purified, if desired, by fractional distillation.

The product treated in the present invention can be produced by the process described in copending Ser. No. 60/151,577. In that process, after disproportionation the solid product consisting of K2NDA isomers is washed and the liquid is filtered to remove catalyst and coke particles.

The liquid carrying mixed organic salts is introduced into a two-stage evaporative crystallization section where the K2NDA is selectively precipitated, KHCO₃ is recycled, and the purified K2NDA is redissolved with additional H₂O. Then the purified K2NDA is passed through an activated carbon bed.

Next, the dipotassium salt of 2,6-NDA, (K2NDA) is selectively precipitated using CO₂ to make the KHNDA solids which are then disproportionated into 2,6 -NDA and K2NDA. The product of disproportionation is centrifuged to yield a 2,6 NDA slurry, and a centrate containing predominantly 2,6 K2NDA and KHCO₃ . The product 2,6-NDA contains from 60-1000 ppm potassium on a dry basis. It is desirable to reduce the amount of potassium to 50 ppm or less.

The key to obtaining polymer grade product with < 50 ppm potassium without using excessive water is the combined use of a 5:1 water wash and a pipeline reactor to drive the reaction to completion and/or remove trace K⁺.

The 2,6-NDA and trace KHNDA are directed into a water wash. In the water wash a ratio of about 3 to 8 parts water is added to one part 2,6-NDA solid and KHNDA and reacted at a temperature of 90 to 180°C, for up to about one hour. Good results were observed washing with about 5 parts water and heating to 150°C for 30 minutes. The higher temperature is preferred because it affords higher solids loading.

The 2,6-NDA and KHNDA are then introduced into a reactor of the type known as a pipeline reactor, characterized by plug flow kinetics. In a plug flow reactor very little, if any, backmixing of product with feed occurs, as contrasted with a stirred vessel reactor or pipe loop reactor. Reactors of the type known in the art as turbulent flow would also be effective.

The temperature in the pipeline reactor should be in the range of 100-200°C. The preferred range is 140-170°C, with a temperature in the range of 150°C providing good results.

The residence time in the pipeline reactor can vazy. The slurry is reacted at plug flow conditions to drive the KHNDA disproportionation reaction toward completion and to remove trace potassium. In example 1 it was found that the desired results were achieved under plug flow conditions in 30 minutes to an hour.

The contents of the pipeline reactor is directed to a centrifuge where trace contaminants are removed and recycled back to the KHNDA disproportionation reactor.

A second water wash is optional, but is included in the preferred embodiment. This second wash makes it possible to reduce the ppms of potassium to less than 50ppm. The 2,6-NDA is combined with water, again at a ratio of 3-8 parts water to one part 2,6-NDA and a temperature of 100° to 200°C. Good results were achieved using a five fold excess water, and increasing the temperature to 150°C. Example 2 demonstrates the reduction of potassium levels to <50 ppm.

The slurry containing the product 2,6-NDA is directed to a last centrifuge to separate the product from the water.

The 2,6-NDA solid can be dried by conventional means, apparent to those skilled in the art, or as described, for example, in U.S. 5,292,934 or 5,840,968. However, where dry handling of the solid product is practiced, particle size control can be critical and the handling of 2,6-NDA particles is difficult and' costly.

As mentioned above, it has previously not been possible in the art to obtain commercially available 2, 6-NDA which is acceptable for polymerization. With the present invention it is now possible to produce 2,6-NDA with less than 50 ppm potassium. Other impurities are not as much of a problesn as in the prior art either using the novel integrated process of copending Ser. No. 60/151,577 which avoids the isolation of purified naphthoic acid using a novel hydrodebromination step.

The process of copending Ser. No. 60/151,577 makes it possible to spring impurities. Therefore, it is now possible to produce 2, 6-NDA with a low level of impurities and less than 50 ppm potassium. 2, 6-NDA of this quality can also be transported in a water slurry which is convenient for close coupling with a process for making polyethylene naphthalate (PEN), thus avoiding the difficulties associated with product particle size control, drying, and solids handling. This is discussed in more detail in U.S. Ser. No. 60/151,603.

The present invention will be more clearly understood from the following examples. It is understood that these examples are presented only to illustrate certain embodiments of the invention and are not intended to limit the scope thereof.

### EXAMPLE 1 (control)

Example 1 describes the method used to reduce the ppms of potassium in the 2,6 NDA solid product. In Example 1first KHNDA was disproportionated and the resulting 2,6 NDA solid product was washed with water in a ratio of 5:1, water to solids, at temperatures of 95°C and 135°C for periods of time as indicated. The levels of potassium were checked and then the solids were reslurried twice to obtain the ppmw of potassium recorded in the last column. This example employs kinetics which are expected in the pipe line reactor with turbulent flow, or a batch reactor, or plug flow reactor. Results are recorded in Table 1:

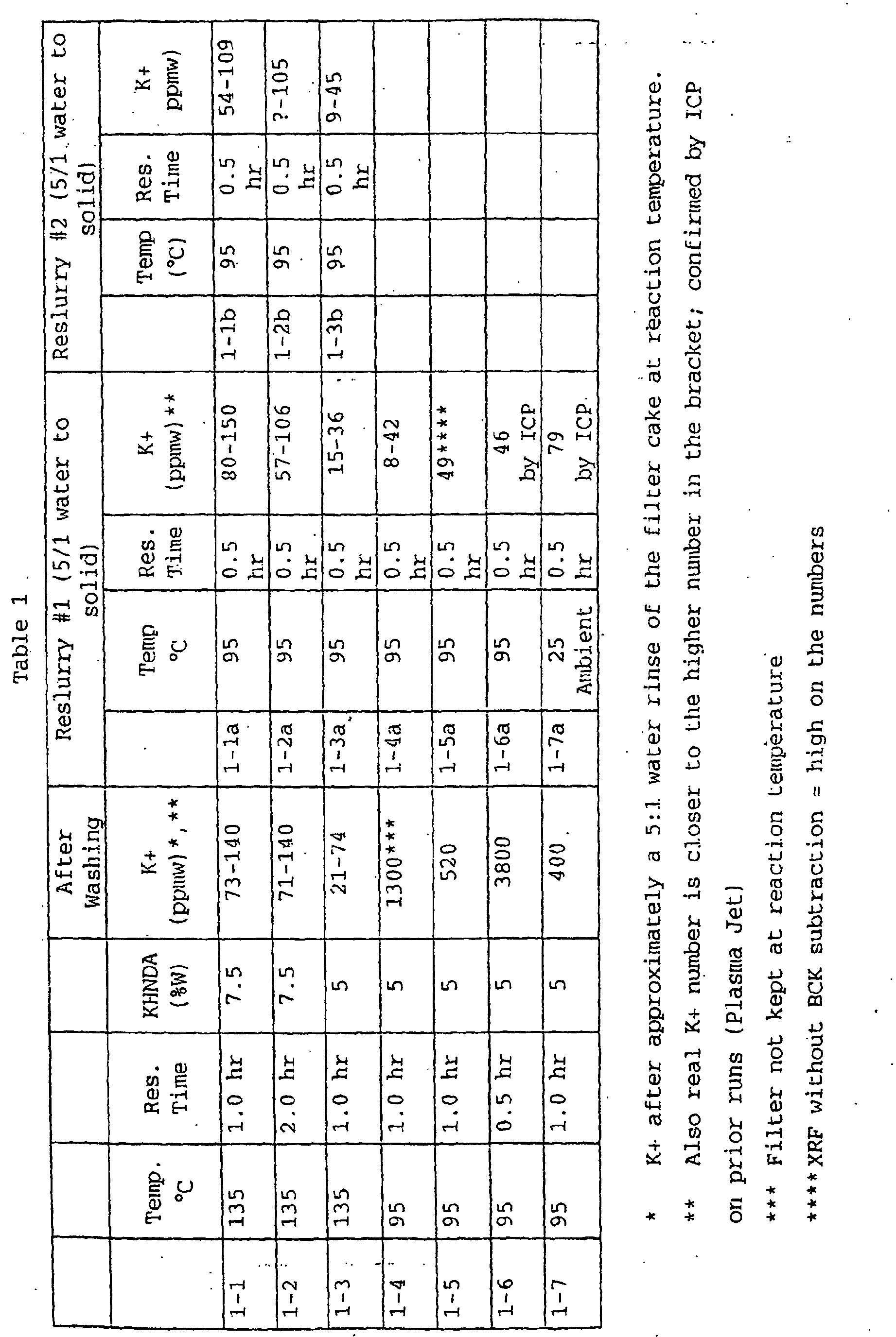

### EXAMPLE 2

Example 2 demonstrates the preferred conditions for purifying the solid 2,6 NDA with increased solids loading. The residence time was about 30 minutes to an hour, the temperature was 150°C, and the disproportionation reaction was assisted by the use of 1.03·10⁷ Pa (150 psig) CO₂. The solids were then washed with water in an 5:1 ratio of water to NDA and left at 150°C for 0.5 hours. The solids were then reslurried once. Results are recorded in Table 2.

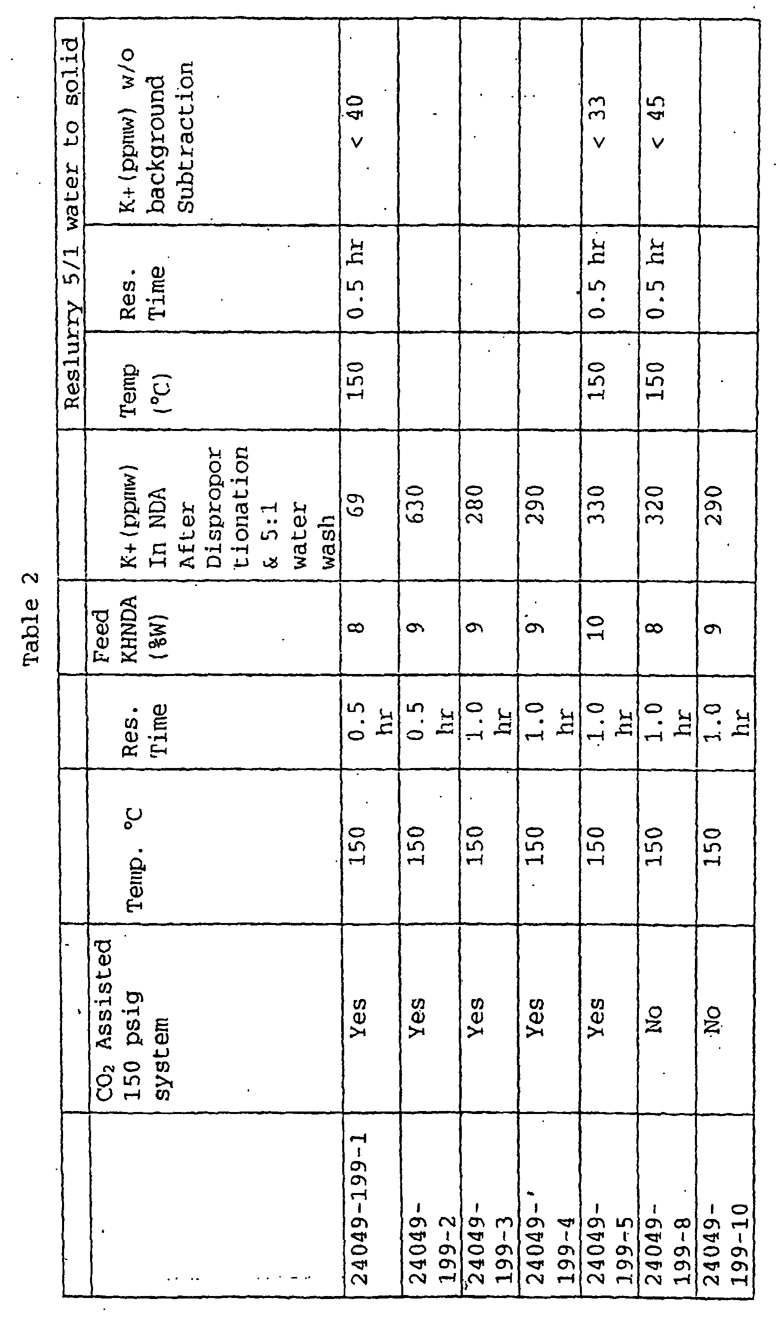

## Claims

1. A method for reducing alkali metals in 2,6-naphthalene dicarboxylic acid produced by disproportionation or rearrangement of an alkali salt of a monocarboxylic acid assisted by CO₂, which comprises:
a) washing the aromatic dicarboxylic acid with water in a ratio of 4-8:1 water to acid at a temperature of 70-200°C;
b) introducing the aromatic dicarboxylic acid in water into a reactor **characterized by** minimal backmixing which is selected from a pipeline reactor and a plug flow type reactor, and reacting the aromatic dicarboxylic acid in water in said reactor at a temperature of 100-200°C;
c) directing the aromatic dicarboxylic acid in water exiting said reactor to a centrifuge to separate the solid aromatic dicarboxylic acid from water containing contaminants.

2. The process of Claim 1 wherein the alkali metal is potassium.

3. The process of Claim 1 further comprising washing the 2,6-naphthalene dicarboxylic acid with water in a ratio of 5 parts water to one part 2,6-NDA at a temperature of 140-160°C; reacting the 2,6-NDA in a pipeline reactor for up to one hour at 140-170°C; and centrifuging the contents exiting the pipeline reactor to separate the 2,6-NDA from water containing contaminants.

4. The process of Claim 3 which further comprises washing the 2,6-NDA a second time, after reacting in the pipe reactor, with water at a ratio of five parts water to one part 2,6-NDA at a temperature of 130-170°C for up to one hour.

5. The process of Claim 4 wherein the amount of potassium in the product 2,6-naphthalene dicarboxylic acid is reduced to less than 50 ppmw.

6. The process of Claim 1, which comprises the further step of combining the solid aromatic dicarboxylic acid again with water in a ratio of 5:1 at a temperature of 100-200°C to further reduce levels of alkali metals.

## Patentansprüche

1. Verfahren zur Verringerung von Alkalimetallen in 2,6-Naphthalindicarbonsäure, die durch Disproportionierung oder Umlagerung eines Alkalisalzes einer Monocarbonsäure mit CO₂-Unterstützung produziert wurde, welches Folgendes umfasst:
a) das Waschen der aromatischen Dicarbonsäure mit Wasser in einem Wasser-Säure-Verhältnis von 4-8:1 bei einer Temperatur von 70-200°C;
b) das Einbringen der aromatischen Dicarbonsäure in Wasser in einen durch minimales Rückvermischen **gekennzeichnet**en Reaktor, der ausgewählt ist aus einem Rohrreaktor und einem idealen Strömungsreaktor, und das Umsetzen der aromatischen Dicarbonsäure in Wasser in diesem Reaktor bei einer Temperatur von 100-200°C;
c) das Leiten der den Reaktor verlassenden, aromatischen Dicarbonsäure in Wasser zu einer Zentrifuge, um die feste aromatische Dicarbonsäure vom Verunreinigungen enthaltenden Wasser zu trennen.

2. Verfahren nach Anspruch 1, wobei das Alkalimetall Kalium ist.

3. Verfahren nach Anspruch 1, weiters umfassend das Waschen der 2,6-Naphthalindicarbonsäure mit Wasser in einem Verhältnis von 5 Teilen Wasser zu einem Teil 2,6-NDA bei einer Temperatur von 140-160°C; das bis zu einstündige Umsetzen der 2,6-NDA in einem Rohrreaktor bei 140-170°C; und das Zentrifugieren der den Rohrreaktor verlassenden Inhalte, um die 2,6-NDA vom Verunreinigungen enthaltenden Wasser zu trennen.

4. Verfahren nach Anspruch 3, welches weiters das nochmalige, bis zu einstündige Waschen der 2,6-NDA mit Wasser in einem Verhältnis von fünf Teilen Wasser zu einem Teil 2,6-NDA bei einer Temperatur von 130-170°C nach dem Umsetzen im Rohrreaktor umfasst.

5. Verfahren nach Anspruch 4, wobei die Menge an Kalium im Produkt 2,6-Naphthalindicarbonsäure auf weniger als 50 ppmw reduziert wird.

6. Verfahren nach Anspruch 1, welches den weiteren Schritt des erneuten Kombinierens der festen aromatischen Dicarbonsäure mit Wasser in einem Verhältnis von 5:1 bei einer Temperatur von 100-200°C umfasst, um die Alkalimetallpegel weiter zu verringern.

## Revendications

1. Procédé pour réduire des métaux alcalins dans de l'acide 2,6-naphtalène dicarboxylique produit par dismutation ou réarrangement d'un sel alcalin d'un acide monocarboxylique assisté par du CO₂, qui comprend :
a) de laver l'acide dicarboxylique aromatique avec de l'eau dans un rapport de l'eau à l'acide de 4 à 8 / 1 à une température de 70 à 200 °C ;
b) d'introduire l'acide dicarboxylique aromatique dans l'eau dans un réacteur **caractérisé par** un mélange à contre-courant minimal qui est choisi parmi un réacteur tubulaire et un réacteur dé type écoulement piston, et de faire réagir l'acide dicarboxylique aromatique dans l'eau dans ledit réacteur à une température de 100 à 200 °C ;
c) de diriger l'acide dicarboxylique aromatique dans l'eau sortant dudit réacteur vers une centrifugeuse afin de séparer l'acide dicarboxylique aromatique solide de l'eau contenant des contaminants.

2. Procédé selon la Revendication 1 dans lequel le métal alcalin est le potassium.

3. Procédé selon la Revendication 1 comprenant en outre de laver l'acide 2,6-naphtalène dicarboxylique avec de l'eau selon un rapport de 5 parties d'eau pour une partie de 2,6-NDA à une température de 140 à 160 °C ; de faire réagir le 2,6-NDA dans un réacteur tubulaire pendant une durée allant jusqu'à une heure à une température de 140 à 170 °C ; et de centrifuger les contenus sortant du réacteur tubulaire pour séparer le 2,6-NDA de l'eau contenant des contaminants.

4. Procédé selon la Revendication 3 qui comprend en outre de laver le 2,6-NDA une seconde fois, après réaction dans le réacteur tubulaire avec de l'eau selon un rapport de cinq parties d'eau pour une partie de 2,6-NDA à une température de 130 à 170 °C pendant une durée allant jusqu'à une heure.

5. Procédé selon la Revendication 4 dans lequel la quantité de potassium dans le produit acide 2,6-naphtalène dicarboxylique est réduite à moins de 50 ppm en poids.

6. Procédé selon la Revendication 1, qui comprend l'étape supplémentaire de combiner l'acide dicarboxylique aromatique solide de nouveau avec de l'eau selon un rapport de 5 / 1 à une température de 100 à 200 °C pour réduire davantage les niveaux de métaux alcalins.
